# EUROPEAN PATENT SPECIFICATION

(11) **EP 1 239 837 B1**
(45) Date of publication and mention of the grant of the patent: **05.01.2005**
(21) Application number: 00972191.1
(22) Date of filing: 13.10.2000
(51) Int. Cl.: A61K 9/20, A61K 31/785

(54) **DIRECT COMPRESSION POLYMER TABLET CORE**
DIREKTKOMPRIMIERTER TABLETTENKERN AUF POLYMERBASIS
NOYAU DE COMPRIME POLYMERE PAR COMPRESSION DIRECTE

(30) Priority: 19.10.1999 US 160258 P; 03.01.2000 US 174227 P
(43) Date of publication of application: 18.09.2002
(73) Proprietor: GENZYME CORPORATION, Cambridge, Massachusetts 02139 (US)
(72) Inventor: TYLER, Joseph, Somerville, MA 02145 (US); PETERSEN, John, S., Acton, MA 01720 (US)
(74) Representative: Kirkham, Nicholas Andrew
(86) International application number: PCT/US2000/028548
(87) International publication number: WO 2001/028527

(56) References cited:
- EP-A- 0 997 148
- WO-A-93/00915
- WO-A-99/22721
- DATABASE CHEMABS [Online] CHEMICAL ABSTRACTS SERVICE, COLUMBUS, OHIO, US; MATSUDA, KATSUYA ET AL: "Tablets containing phosphate-binding polymers and manufacture thereof" retrieved from STN Database accession no. 132:284260 CA XP002165576 & WO 00 22008 A (CHUGAI SEIYAKU KABUSHIKI KAISHA, JAPAN) 20 April 2000 (2000-04-20)

## Description

### BACKGROUND OF THE INVENTION

A number of polymeric materials having useful therapeutic activity have been described for treatment of various conditions such as hyperlipidemia and hyperphosphatemia. Many of these polymeric materials function as non-absorbed ion exchange resins in the digestive tract. Such non-absorbed polymeric materials bind or otherwise sequester a target molecule and facilitate its removal from the body via the gastrointestinal tract. Examples of such resins include: Colestipol and Cholestyramine useful as orally administered cholesterol lowering agents; a variety of aliphatic amine polymers disclosed U.S. Patent Nos 5,496,545 and 5,667,775 useful as phosphate binders particularly for removing phosphate from patients suffering from renal failure; and other aliphatic amine polymers disclosed in U.S Pat No. 5,624,963, U.S. Pat No. 5,679,717, WO98/29107 and WO99/22721 useful as cholesterol lowering agents.

Non-absorbed polymer therapeutics have traditionally presented a number of formulation challenges as the dosages are generally very large (gram quantities), and the resins tend to be extremely hydrophilic. The most desirable formulation for oral delivery of a therapeutic is a direct compression tablet formulation. However, not all therapeutics, particularly given the high dose requirements of polymeric ion exchange therapeutics, lend themselves to a tablet formulation. Even if such materials could be rendered into a tablet, it is generally not possible without the significant addition of other materials which assist in the tableting process. Ultimately the addition of any materials other than the active ingredient is undesirable given the dose requirement of the active ingredient. Ideally the tablet should contain as much active ingredient as possible with little else in the way of additional materials such that the tablet is as small as possible and easy to administer to the patient.

In addition, once the polymeric materials are compressed into a tablet, the tablet requires a coating for ease of administration to the patient. It has been discovered that the core polymeric material tends to be very hygroscopic, and thus will swell immediately upon contact with the inside of the mouth. Most coatings contain water, and thus it was believed that coating such tablets with a water-based coating would be impossible because the hygroscopic tablets would swell during the coating process. Thus providing a tablet core comprising a hygroscopic material such that a suitable coating may be used in conj unction with that core, is another significant challenge to providing the polymeric active ingredient in tablet form.

There is a need to provide suitable dosage forms for polymeric ion exchange materials, particularly for hydrophilic aliphatic amine polymers useful as therapeutic agents, which minimize the overall amount of material administered to the patient, which are easy to administer orally, and which are stable upon production and storage.

### SUMMARY OF THE INVENTION

The present invention provides a tablet with a tablet core which comprises at least about 95% by weight of an aliphatic amine polymer. In a preferred embodiment, the aliphatic amine polymer resin is a cross-linked polyallylamine resin. The aliphatic amine polymer is preferably hydrated. The hydrated polymer can, for example, comprise from about 5% water by weight or greater.

The tablet of the invention may have the tablet core provided by a method of producing a tablet core comprising at least about 95% by weight of an aliphatic amine polymer resin. The method comprises the step of compressing the aliphatic amine polymer to form the tablet core. The tablet core can further include one or more excipients. In this embodiment, the method of producing the tablet core comprises the steps of: (1) hydrating or drying the aliphatic amine polymer to the desired moisture level; (2) blending the aliphatic amine polymer with the excipients in amounts such that the polymer comprises at least about 95% by weight of the resulting blend; and (3) compressing the blend to form the tablet core.
The present invention further relates to a coated tablet wherein the coating comprises a water based coating.

### BRIEF DESCRIPTION OF THE DRAWINGS

The Figure is a table comprising data showing formulations and responses for sevelamer hydrochloride compressed tablet cores.

### DETAILED DESCRIPTION OF THE INVENTION

A number of polymeric materials having useful therapeutic activity have been discussed above. In particular, aliphatic amine polymers have been disclosed which are useful in methods of lowering the serum phosphate level of a patient and lowering the serum cholesterol level of a patient. For example an epichorohydrin-cross-linked poly(allylamine hydrochloride) resin (U.S. Patent Nos. 5,496,545 and 5,667,775), also referred to as sevelamer hydrochloride or sevelamer and marketed as Renagel®, has been shown to be effective at removing phosphate from human patients suffering from renal failure. Therapeutically effective dosages of sevelamer hydrochloride are large, typically on the order of 4 to 6 grams per day. Consequently, development of a dosage form of this and similar resins which minimizes the amount of excipient material is desirable.

The present invention provides a tablet with a tablet core comprising at least about 95% by weight of an aliphatic amine polymer. The aliphatic amine polymer resin can be any of the aliphatic amine resins described in U.S. Patent Nos. 5,496,545; 5,667,775; 5,624,963; 5,703,188; 5,679,717; 5,693,675, 5,607,669; 5,618,530; 5,487,888; and 5,702,696. Other suitable aliphatic amine polymers are disclosed in US 6034129, US 5840766 and US 6423754. In a particularly preferred embodiment, the aliphatic amine polymer is polyallylamine, polyvinylamine, poly(diallylamine) or poly(ethyleneimine) or a salt thereof with a pharmaceutically acceptable acid. The aliphatic amine polymer is optionally substituted at one or more nitrogen atoms with an alkyl group or a substituted alkyl group such as a trialkylammonioalkyl group. The aliphatic amine polymer can optionally be cross-linked, for example via a multifunctional monomer or a bridging group which connects two amino nitrogen atoms from two different polymer strands.
In a preferred embodiment, the aliphatic amine polymer resin is hydrated. For sevelamer hydrochloride, in particular, the compressibility is strongly dependent upon the degree of hydration (moisture content) of the resin. Preferably, the resin has a moisture content of about 5% by weight or greater, more preferably, the moisture content is from about 5% to about 9% by weight, and most preferably about 7% by weight. It is to be understood that in embodiments in which the polymer resin is hydrated, the water of hydration is considered to be a component of the resin. Thus, in this embodiment, the tablet core comprises at least about 95%, preferably at least about 96%, and more preferably at least about 98% by weight of the hydrated polymer, including the water of hydration.

The tablet can further comprise one or more excipients, such as hardeners, glidants and lubricants, which are well known in the art. Suitable excipients include colloidal silicon dioxide, stearic acid, magnesium silicate, calcium silicate, sucrose, calcium stearate, glyceryl behenate, magnesium stearate, talc, zinc stearate and sodium stearylfumarate. The excipients can represent from 0 to about 5% of the tablet core by weight.

The tablet core of the tablet of the invention is prepared by a method comprising the steps of: (1) hydrating or drying the aliphatic amine polymer to the desired moisture level;
(2) blending the aliphatic amine polymer with any excipients to be included in amounts such that the polymer comprises at least about 95% by weight of the resulting blend; and (3) compressing the blend using conventional tableting technology.

The invention also relates to a stable, swallowable coated tablet, particularly a tablet comprising a hydrophilic core, such as a tablet comprising an aliphatic amine polymer, as described above. In one embodiment, the coating composition comprises a cellulose derivative and a plasticizing agent. The cellulose derivative is, preferably, hydroxypropylmethylcellulose (HPMC). The cellulose derivative can be present as an aqueous solution. Suitable hydroxypropylmethylcellulose solutions include those containing HPMC low viscosity and/or HPMC high viscosity. Additional suitable cellulose derivatives include cellulose ethers useful in film coating formulations. The plasticizing agent can be, for example, an acetylated monoglyceride such as diacetylated monoglyceride, The coating composition can further include a pigment selected to provide a tablet coating of the desired color. For example, to produce a white coating, a white pigment can be selected, such as titanium dioxide.

In one embodiment, the coated tablet of the invention can be prepared by a method comprising the step of contacting a tablet core produced as described above, with a coating solution comprising a solvent, at least one coating agent dissolved or suspended in the solvent and, optionally, one or more plasticizing agents. Preferably, the solvent is an aqueous solvent, such as water or an aqueous buffer, or a mixed aqueous/organic solvent. Preferred coating agents include cellulose derivatives, such as hydroxypropylmethylcellulose. Typically, the tablet core is contacted with the coating solution until the weight of the tablet core has increased by an amount ranging from about 4% to about 6%, indicating the deposition of a suitable coating on the tablet core to form a coated tablet.

In one preferred embodiment, the solids composition of the coating solution is:

| Material | %W/W |
|---|---|
| HPMC low viscosity Type 2910, cUSP | 38.5% |
| HPMCE high viscosity Type 2910, cUSP | 38.5% |
| diacetylated monoglyceride | 23.0% |

Tablets may be coated in a rotary pan coater as is known in the art or any other conventional coating apparatus such as a column coater or a continuous coater.

Astonishingly, it has been found that an aqueous coating dispersion is suitable as a coating solution for tablets comprising a hygroscopic, or water-swellable substance, such as an aliphatic amine polymer tablet. For example, the coating composition provides a strong, elastic and moisture-permeable coating without causing significant concomitant swelling of the tablet core during the coating process. In a preferred embodiment, the coating composition provides a tablet coating which withstands the swelling and contraction of sevelamer hydrochloride tablets during exposure to varying humidity levels and other known stability tests. Further, the coating composition can be used to coat other aliphatic amine polymer tablets without excessive uptake by the tablet core of water from the coating solution during the coating process.

The present invention also relates to the use of an aliphatic amine polymer as a disintegrant in a tablet. In general, in this embodiment the aliphatic amine polymer is not the active ingredient in the tablet, but is added to the tablet to enhance the rate of disintegration of the tablet following administration. This allows a more rapid release of the active agent or agents. The tablet will generally include the aliphatic amine polymer, one or more active ingredients, such as therapeutic agents (medicaments), and, optionally, one or more additional excipients.

The aliphatic amine polymer can be one of the aliphatic amine polymers disclosed above, such as polyethyleneimine, polyvinylamine, polyallylamine, polydiallylamine or any of the aliphatic amine polymers disclosed in U.S. Patent Nos. 5,496,545, 5,667,775, 6 203 785 and 6 726 905. In one embodiment, the aliphatic amine polymer is a cross-linked polyallylamine or a salt thereof with a pharmaceutically acceptable acid. Preferably, the aliphatic amine polymer is an epichlorohydrin-cross-linked polyallylamine or salt thereof with a pharmaceutically acceptable acid, such as sevelamer or sevelamer hydrochloride.

The tablet which includes an aliphatic amine as a disintegrant will, generally, include a sufficient amount of the aliphatic amine polymer to effectively enhance the rate of tablet disintegration under conditions of use. For example, if the tablet is an oral doseage form and it is desired that the tablet disintegrate in the stomach of the patient, the tablet should include a sufficient amount of the polymer to enhance the disintegration rate of the tablet under the conditions encountered in the stomach. The appropriate amount of the polymer to be included in the tablet can be determined by one skilled in the art using known methods. Typically, the polymer, the active ingredient or ingredients and any additional fillers or excipients are combined by mixing, and the resulting mixture is compressed to form a tablet using conventional methods. The tablet core formed in this way can then be coated, for example, as described above, or by other methods and other coating compositions which are known in the art and suitable for the intended use of the tablet.

In one embodiment, the tablet which includes an aliphatic amine polymer as a disintegrant is intended for administration *in vivo,* for example, to a patient, such as a human. Preferably, the tablet is intended to be administered orally. In this embodiment, the active ingredient or ingredients will be a therapeutic or diagnostic agent. The tablet can also be intended for use *in vitro*, for example, to deliver an active ingredient to an aqueous environment, such as a swimming pool.

The invention will now be described in detail by reference to the following examples.

### EXAMPLES

### Example 1 Preparation and characterization of 400 mg and 800 mg sevelamer hydrochloride direct compression tablet cores.

### Preparation of tablet cores

400 mg sevelamer hydrochloride tablet cores were prepared from a blend consisting of 5000.0 g sevelamer hydrochloride, 50.0 g colloidal silicon dioxide, NF (Aerosil 200) and 50.0 g stearic acid. The sevelamer hydrochloride was hydrated to moisture content of 6% by weight. The blend was prepared by passing the sevelamer hydrochloride and colloidal silicon dioxide through a #20 mesh screen, transferring the mixture to a 16 quart PK blender and blending for five minutes. The stearic acid was then passed through an oscillator equipped with a #30 mesh screen, transferred into the 16 quart PK blender and blended for five minutes with the sevelamer hydrochloride/ colloidal silicon dioxide mixture. The resulting blend was discharged into a drum and weighed. The final blend was then compressed on a 16 station Manesty B3B at 4 tons pressure using 0.280" x 0.620" punches to give tablet cores with an average weight of 434 mg. The resulting tablets consisted of 425 mg 6% hydrated sevelamer hydrochloride (equivalent to 400 mg anhydrous sevelamer hydrochloride), 4.25 mg colloidal silicon dioxide and 4.25 mg stearic acid.

800 mg sevelamer hydrochloride tablet cores were prepared from 19.0 kg sevelamer hydrochloride, 0.19 kg colloidal silicon dioxide, and 0.19 kg stearic acid,. The sevelamer hydrochloride had a moisture content of 6% by weight. The blend was prepared by passing the sevelamer hydrochloride and colloidal silicon dioxide through a #20 mesh screen, transferring the mixture to a PK blender and blending for five minutes. The stearic acid was then passed through an oscillator equipped with a #30 mesh screen, transferred into the PK blender and blended for five minutes with the sevelamer hydrochloride/colloidal silicon dioxide mixture. The resulting blend was then discharged into a drum and weighed. The final blend was then compressed in on a 16 station Manesty B3B at 4 tons pressure using 0.3125" x 0.750" punches to give tablets with an average weight of 866 mg. The resulting tablets consisted of 850 mg 6% hydrated sevelamer hydrochloride (equivalent to 800 mg anhydrous sevelamer hydrochloride), 8.0 mg colloidal silicon dioxide and 8.0 mg stearic acid.

### Characterization of tablet cores

The tablets prepared as described above were white to off-white, oval shaped, compressed tablets. The variation of the tablets prepared from each blend with respect to weight, thickness, friability, hardness, disintegration time and density was assessed. Standard methods in the art were employed for each of the measurements. The results, (not shown), indicate that the hardness, friability, thickness, and disintegration behavior of the sevelamer hydrochloride tablets all met industry-standard criteria.

### Example 2 Coating of sevelamer hydrochloride tablet cores.

Compressed core tablets prepared as described in Example 1 were coated in a coating pan with an aqueous coating solution having a solids composition comprising:

| Material | %W/W |
|---|---|
| HPMC low viscosity Type 2910, cUSP | 38.5% |
| HPMCE high viscosity Type 2910, cUSP | 38.5% |
| diacetylated monoglyceride | 23.0% |

The coating solution was applied to the compressed cores until a weight gain of approximately 4 to 6% was achieved. Stability studies - controlled room temperature, accelerated conditions, freeze/thaw and photosensitivity - for the coated sevelamer hydrochloride tablets were conducted in accordance with those procedures known in the art and described in the following references: International Committee on Harmonization (ICH) guidance "Q1A-Stability Testing of New Drug Substances and Products" (June 1997); ICH "Q1B-Guidelines for the Photostability Testing of New Drug Substances and Products" (November 1996);and ICH guidance "Q1C-Stability Testing for New Dosage Forms" (November 1996. The results (not shown) indicate that the coated tablets all met industry standard criteria.

### Example 3 Factors affecting the Processing and Performance Characteristics of Compressed Tablets (prior to coating)

In order to maintain consistently acceptable compressed tablet on a per batch basis, a number of correlative tests were performed in order to determine which factors most strongly impact the quality and integrity of the tablets. Studies such as weight variation, tablet hardness, friability, thickness, disintegration time, among others are known to those skilled in the art and are described in the United States Pharmacopeia (U.S.P.). "Hardness" means the measure of the force (measured herein in Newtons) needed to fracture a tablet when such tablet is placed lengthwise on a Hardness Tester. "Friability" is the measure of the mechanical strength of the tablet needed to withstand the rolling action of a coating pan and packaging. It is measured using a friabiliator. "Thickness" is the measure of the height of the tablet using a micrometer. "Disintegration Time" is the time necessary for the tablet to break apart in an appropriate solution at 37° C and is measured in minutes.

Attainment of appropriate hardness (150-170 N hardness range) and friability (no more than 0.8%) is important to the success of the formulation. Having tablets with high hardness and low friability is particularly important when the tablets are to be coated as is the case with sevelamer hydrochloride tablets

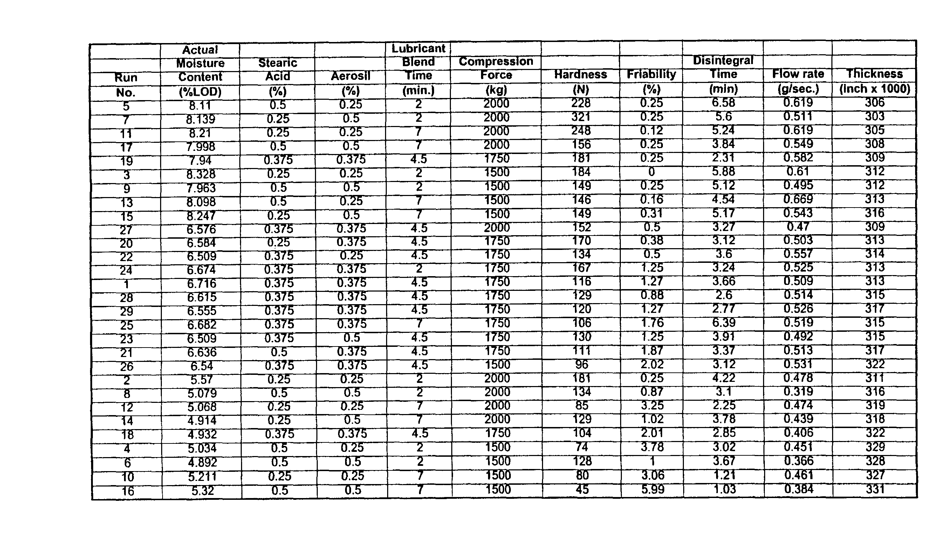

Fig. 1 provides a table listing several different sevelamer hydrochoride tablet core formulations that vary by a number of factors including (actual) moisture content, and compression force used, excipient content among other variations. The data in

Fig. 1 indicates that the most important factor affecting the processing and performance characteristics of compressed tablets is the moisture content. All formulations provided good flow with little weight variation throughout the entire range of compositions. In addition, disintegration times were less than 5 minutes across the range of compositions. Thus, it appears that moisture content and compression force provide the most appropriate factors on which to establish operating ranges for hardness and friability.

## Claims

1. A tablet comprising a core and coating therefor, wherein at least about 95% by weight of the core is an aliphatic amine polymer selected from the group consisting of unsubstituted and N-substituted poly(allylamine), poly(diallylamine) and poly(vinylamine).

2. The tablet of Claim 1, wherein (a) the N-substituents are selected from the group consisting of susbtituted and unsubstituted C₁-C₂₄-alkyl groups (b); the aliphatic amine polymer is cross-linked (c); the core further comprises one or more excipients (d); the coating is a water-based coating; and/or (e) said polymer is polydiallylamine.

3. A tablet comprising a core and a coating therefor, wherein at least about 95% by weight of the core is a linear or cross-linked poly(allylamine) or a pharmaceutically acceptable salt thereof.

4. The tablet of Claim 3, wherein (a) the poly(allylamine) is hydrated; and optionally wherein the poly(allylamine) comprises from about 3% to about 10% water; and further optionally wherein the poly(allylamine) comprises from about 5% to about 8% water; and further optionally wherein the polyallylamine is from about 1% to about 10% cross-linked; (b) at least about 95% by weight of the core is a hydrated cross-linked poly(allylamine hydrochloride); or (c) the coating is a water-based coating; and optionally wherein said water-based coating comprises hydroxypropylmethylcellulose and a plasticizer; and further optionally wherein said coating comprises hydroxypropyl methylcellulose low viscosity, hydroxypropylmethylcellulose high viscosity, and diacetylated monoglyceride.

5. A compressed tablet comprising a pharmaceutically active agent and an effective disintegrating amount of polyallylamine or a salt thereof with a pharmaceutically acceptable acid.

6. The compressed tablet of Claim 5 wherein the polyallylamine or salt thereof is cross-linked; and optionally wherein the polyallylamine or salt thereof is cross-linked with epichlorohydrin.

7. A tablet comprising a core and a coating therefor, wherein the core comprises 98% by weight sevelamer hydrochloride with a moisture content of 6% by weight, 1% by weight colloidal silicon dioxide and 1% by weight stearic acid, and wherein the coating is a mixture comprising 38.5% w/w low viscosity hydropropylmethyl celluose, 38.5% w/w high viscosity hydropropylmethyl celluose and 23% w/w diacetylated monoglyceride.

## Patentansprüche

1. Tablette, umfassend einen Kern und einen Überzug dafür, wobei mindestens etwa 95 Gew.-% des Kerns ein aliphatisches Amin-Polymer sind, das aus der Gruppe ausgewählt ist, bestehend aus unsubstituiertem und N-substituiertem Poly(allylamin), Poly(diallylamin) und Poly(vinylamin).

2. Tablette nach Anspruch 1, wobei (a) die N-Substituenten aus der Gruppe ausgewählt sind, bestehend aus substituierten und unsubstituierten C₁-C₂₄-Alkylgruppen, (b) das aliphatische Amin-Polymer vernetzt ist; (c) der Kern weiterhin einen oder mehrere Arzneimittelträgerstoffe umfasst; (d) der Überzug ein auf Wasser basierender Überzug ist; und/oder (e) das Polymer Polydiallylamin ist.

3. Tablette, umfassend einen Kern und einen Überzug dafür, wobei mindestens etwa 95 Gew.-% des Kerns ein lineares oder vernetztes Poly(allylamin) oder ein pharmazeutisch verträgliches Salz davon sind.

4. Tablette nach Anspruch 3, wobei
(a) das Poly(allylamin) hydratisiert ist; und wobei das Poly(allylamin) gegebenenfalls etwa 3 bis etwa 10 % Wasser umfasst; und wobei das Poly(allylamin) außerdem gegebenenfalls etwa 5 bis etwa 8 % Wasser umfasst; und wobei das Poly(allylamin) außerdem gegebenenfalls zu etwa 1 bis etwa 10 % vernetzt ist;
(b) mindestens etwa 95 Gew.-% des Kerns ein hydratisiertes vernetztes Poly(allylaminhydrochlorid) sind; oder
c) der Überzug ein auf Wasser basierender Überzug ist; und wobei der auf Wasser basierende Überzug Hydroxypropylcellulose und einen Weichmacher umfasst; und wobei weiterhin der Überzug gegebenenfalls niederviskose Hydroxypropylmethylcellulose, hochviskose Hydroxypropylmethylcellulose und diacetyliertes Monoglycerid umfasst.

5. Komprimierte Tablette, umfassend einen pharmazeutischen Wirkstoff und eine wirksame zerfallsfördernde Menge von Polyallylamin oder einem Salz davon mit einer pharmazeutisch verträglichen Säure.

6. Komprimierte Tablette nach Anspruch 5, wobei das Polyallylamin oder das Salz davon vernetzt ist; und wobei das Polyallylamin oder das Salz davon gegebenenfalls mit Epichlorhydrin vernetzt ist.

7. Tablette, umfassend einen Kern und einen Überzug dafür, wobei der Kern 98 Gew.-% Sevelamerhydrochlorid mit einem Feuchtigkeitsgehalt von 6 Gew.-%, 1 Gew.-% kolloidales Siliciumdioxid und 1 Gew.-% Stearinsäure umfasst und wobei der Überzug ein Gemisch ist, umfassend 38,5 % Gew./Gew. niederviskose Hydroxypropylmethylcellulose, 38,5 % Gew./Gew. hochviskose Hydroxypropylmethylcellulose und 23 % Gew./Gew. diacetyliertes Monoglycerid.

## Revendications

1. Tablette comprenant un coeur et un revêtement, dans lequel au moins environ 95% en poids du coeur est un polymère amine aliphatique choisi dans le groupe consistant en la poly(allylamine), la poly(diallylamine), et la poly(vinylamine), non substituées et N-substituées.

2. Tablette selon la revendication 1, dans lequel : (a) les N-substituants sont choisis dans le groupe consistant en les groupes alkyle en C₁-C₂₄ substitués et non substitués ; (b) le polymère amine aliphatique est réticulé ; (c) le coeur comprend en outre un ou plusieurs excipients ; (d) le revêtement est un revêtement à base d'eau ; et/ou (e) ledit polymère est une polydiallylamine.

3. Tablette comprenant un coeur et un revêtement, dans lequel au moins environ 95% en poids du coeur est une poly(allylamine) réticulée ou linéaire, ou un de ses sels pharmaceutiquement acceptables.

4. Tablette selon la revendication 3, dans lequel (a) : la poly(allylamine) est hydratée ; la poly(allylamine) comprenant éventuellement d'environ 3% à environ 10% d'eau ; et où la poly(allylamine) comprend en outre, de façon optionnelle d'environ 5% à environ 8% d'eau ; et où la polyallylamine est éventuellement réticulée à raison d'environ 1% à environ 10% ; (b) le coeur est, pour au moins environ 95% en poids, un poly(hydrochlorure d'allylamine) réticulé hydraté ;
ou bien (c) le revêtement est un revêtement à base d'eau ; et ledit revêtement à base d'eau comprenant éventuellement de l'hydroxypropylméthylcellulose et un plastifiant ; et dans lequel ledit revêtement comprend éventuellement en outre de l'hydroxypropylméthylcellulose de faible viscosité, de l'hydroxypropylméthylcellulose à haute viscosité, et un monoglycéride diacétylé.

5. Tablette sous forme de comprimé, comprenant un agent pharmaceutiquement actif et une quantité désintégrante efficace d'un polyallylamine ou d'un de ses sels avec un acide pharmaceutiquement acceptable.

6. Tablette sous forme de comprimé selon la revendication 5, dans lequel la polyallylamine ou son sel est réticulé ; la polyallylamine ou son sel étant éventuellement réticulé avec de l'épichlorhydrine.

7. Tablette comprenant un coeur et un revêtement, dans lequel le noyau comprend 98% en poids d'hydrochlorure de sevelamer ayant une teneur en humidité de 6% en poids, 1% en poids de dioxyde de silicium colloïdal et 1% en poids d'acide stéarique, et dans lequel le revêtement est un mélange comprenant 38,5% poids/poids d'hydropropylméthylcellulose de faible viscosité, 38,5% poids/poids d'hydropropylméthylcellulose de haute viscosité et 23% poids/poids de monoglycéride diacétylé.
